# EUROPEAN PATENT APPLICATION

(11) **EP 4 835 474 A1**
(43) Date of publication of application: **04.11.2026**
(21) Application number: 24911891.0
(22) Date of filing: 27.09.2024
(51) Int. Cl.: G01N 33/543, G01N 33/531, G01N 33/553

(54) **POLYMER-COATED MAGNETIC METAL PARTICLE AND METHOD FOR PRODUCING SAME**

(30) Priority: 26.12.2023 JP 2023218980; 25.09.2024 JP 2024166726
(71) Applicant: DOWA ELECTRONICS MATERIALS CO., LTD., Chiyoda-ku Tokyo 101-0021 (JP)
(72) Inventor: GANNYO Ikumi, Tokyo 101-0021 (JP); NAGATOMI Akira, Tokyo 101-0021 (JP); HONMA Sakura, Tokyo 101-0021 (JP)
(74) Representative: Müller-Boré & Partner Patentanwälte PartG mbB
(86) International application number: PCT/JP2024/034624
(87) International publication number: WO 2025/141997

(57) **Abstract**

A polymer-coated magnetic metal particle according to the present disclosure includes: a magnetic metal particle; a coating layer which is provided on a surface of the magnetic metal particle and is constituted from silicon oxide; and a polymer layer which is provided on a surface of the coating layer and includes a polymer of a structural unit represented by formula (1) and alkoxysilane having an acrylic group or a methacrylic group.

## Description

### Technical Field

The present disclosure relates to a polymer-coated magnetic metal particle and a method for producing same.

### Background Art

In the field of medical diagnostics, various target substances such as proteins, nucleic acids, or cells may be separated and recovered from sample liquids such as blood for testing. As an example of a method for such testing, carrier substances suitable for each target substance are carried on the surface of a predetermined particle, and after capturing the target substance, the particle is recovered and analyzed.

Particles having magnetic properties may be used as particles carrying the carrier substances. Magnetic particles allow the carrier substances to be carried, and after capturing the target substances, the target substance can be recovered by applying an external magnetic field.

As such magnetic particles, for example, magnetic polymer particles have been suggested in which the surface of a core polymer particle is sequentially coated with a coating layer containing nano magnetic material and silicon oxide, and a polymer layer capable of binding the carrier substances (see, for example, Patent Document 1).

### Citation List

### Patent Document

Patent Document 1: Japanese Patent No. 2021-60339 A

### Summary of Invention

### Technical Problem

For the above test particles, from the perspective of improving testing accuracy, it is important that the test particles can carry a large amount of carrier substances capable of binding to target substances. In addition, the test particles are required to have high saturation magnetization and high magnetic responsiveness, that is, to rapidly aggregate when a magnetic field is applied.

In this regard, even if the particles of Patent Document 1 achieve a large carried amount, the saturation magnetization tends to be low because the core is a polymer particle, and the desired magnetic responsiveness may not be obtained.

As such, the present disclosure aims to provide a technique for increasing the carried amount of the carrier substances capable of binding to the target substance while also ensuring that the magnetic responsiveness reaches a certain level or higher.

### Solution to Problem

A first aspect of the present disclosure is a polymer-coated magnetic metal particle including: a magnetic metal particle; a coating layer which is provided on a surface of the magnetic metal particle and is constituted from silicon oxide; and a polymer layer which is provided on a surface of the coating layer and includes a polymer of a structural unit represented by formula (1) and alkoxysilane having an acrylic group or a methacrylic group. 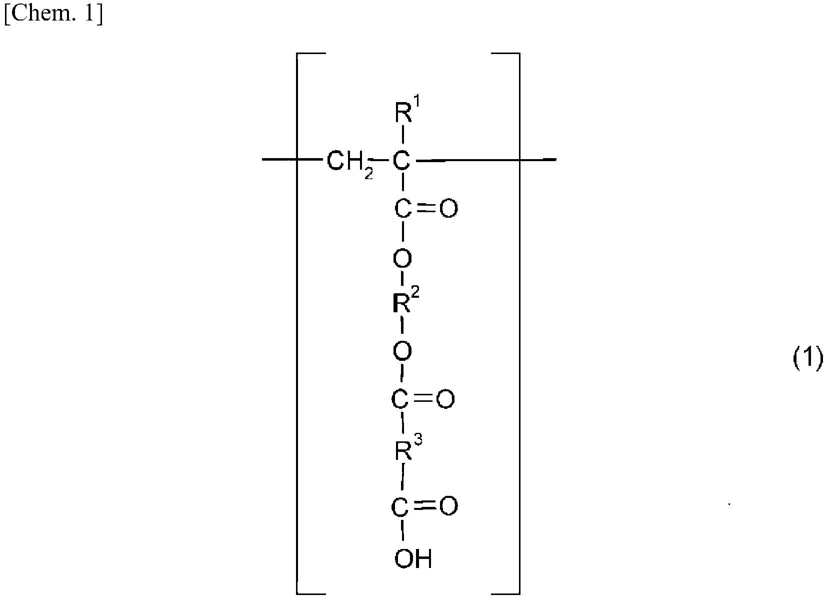 (in formula (1), R¹ represents a hydrogen atom or a methyl group, R² represents a linear or branched alkylene group having 2 or more and 6 or less carbon atoms, and R³ represents an alkylene group having 2 or more and 6 or less carbon atoms, a cyclohexylene group or a phenylene group).

A second aspect of the present disclosure is the first aspect, wherein, in formula (1), R² is an ethylene group, and R³ is an alkylene group, a phenylene group or a cyclohexylene group.

A third aspect of the present disclosure is the second aspect, wherein, in formula (1), R³ is a phenylene group.

A fourth aspect of the present disclosure is any one of the first to third aspects, wherein the alkoxysilane has an acrylic group or a methacrylic group and an alkylene group having 3-6 carbon atoms.

A fifth aspect of the present disclosure is the fourth aspect, wherein the alkoxysilane has an acryloxypropyl group or a methacryloxypropyl group.

A sixth aspect of the present disclosure is any one of the first to fifth aspects, wherein the magnetic metal particle is an iron particle or an iron-based alloy particle.

A seventh aspect of the present disclosure is any one of the first to sixth aspects, wherein saturation magnetization is100 Am²/kg or more and 210 Am²/kg or less.

An eighth aspect of the present disclosure is any one of the first to seventh aspects, wherein a 50% cumulative particle size by volume measured by a laser diffraction particle size distribution analyzer is 0.2-10 µm.

A ninth aspect of the present disclosure is any one of the first to eighth aspects, wherein carbon content is 0.5 mass% or more and 10 mass% or less.

A tenth aspect of the present disclosure is any one of the first to ninth aspects, wherein carbon content is 0.1 mass% or more and 10 mass% or less.

An eleventh aspect of the present disclosure is a method of producing a polymer-coated magnetic metal particle, the method including: forming a coating layer constituted from silicon oxide on a surface of a magnetic metal particle; and mixing the magnetic metal particle on which the coating layer is formed, water, and alkoxysilane having an acrylic group or a methacrylic group to obtain a mixture, then adding a compound represented by formula (2) to the mixture, and polymerizing the mixture to which the compound is added to form a polymer layer on the coating layer. 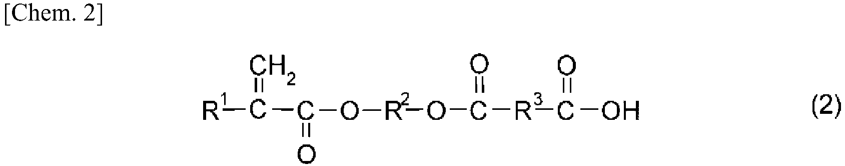 (in formula (2), R¹ represents a hydrogen atom or a methyl group, R² represents a linear or branched alkylene group having 2 or more and 6 or less carbon atoms, and R³ represents an alkylene group having 2 or more and 6 or less carbon atoms, a cyclohexylene group or a phenylene group).

A twelfth aspect of the present disclosure is the eleventh aspect, wherein, in formula (2), R² is an ethylene group, and R³ is an alkylene group, a phenylene group or a cyclohexylene group.

A thirteenth aspect of the present disclosure is the twelfth aspect, wherein, in formula (2), R³ is a phenylene group.

A fourteenth aspect of the present disclosure is any one of the eleventh to thirteenth aspects, wherein the alkoxysilane has: an acrylic group or a methacrylic group; and an alkylene group having 3-6 carbon atoms.

A fifteenth aspect of the present disclosure is the fourteenth aspect, wherein the alkoxysilane has an acryloxypropyl group or a methacryloxypropyl group.

A sixteenth aspect of the present disclosure is any one of the eleventh to fifteenth aspects, wherein the magnetic metal particle is an iron particle or an iron-based alloy particle.

A seventeenth aspect of the present disclosure is any one of the eleventh to sixteenth aspects, wherein, in the forming of the polymer layer, a water-soluble azo polymerization initiator having a carboxyl group is used as a polymerization initiator.

### Advantage of the Invention

The present disclosure allows the test particle to carry a larger amount of carrier substances capable of binding to the target substance while ensuring that the magnetic responsiveness of the test particle reaches a certain level or higher.

### Description of Embodiments

### <One Embodiment of the Present Disclosure>

Hereinafter, a polymer-coated magnetic metal particle and method for producing same according to one embodiment of the present disclosure will be described. In this specification, numerical ranges represented by a hyphen "-" indicate ranges including the numerical values before and after the hyphen "-" as the lower and upper limits, respectively.

### (1) Polymer-Coated Magnetic Metal Particle

The polymer-coated magnetic metal particle in the present embodiment is configured such that a coating layer and a polymer layer are laminated in this order on the surface of a magnetic metal particle. Hereinafter, the polymer-coated magnetic metal particle is also simply referred to as a test particle.

### (Magnetic Metal Particle)

The magnetic metal particle serves as the core of the test particle and is a ferromagnetic particle constituted from a metal or an alloy. Examples of magnetic metal particles constituted from pure metal include iron particles, nickel particles, or cobalt particles, and examples of magnetic metal particles constituted from an alloy include iron-based alloy particles. In this specification, "iron-based alloy" refers to an alloy containing 50 mass % or more of iron, and examples of iron-based alloy particles include Fe-B-based alloy particles, Fe-Si-based alloy particles, Fe-N-based alloy particles, Fe-Ni-based alloy particles, and Fe-C-based alloy particles. From the viewpoint of ensuring excellent saturation magnetization of the test particle, it is preferable to use an iron particle or an iron-based alloy particle as the magnetic metal particle.

The particle size of the magnetic metal particle is preferably 0.2-10.0 _{[}tm. The test particles including such magnetic metal particles can, when added to a sample solution, balance the weight and buoyancy of the test particles and achieve appropriate dispersibility in the sample solution. As a result, adhesion of the carrier substances to the test particle and binding of the target substance to the carrier substances can be more reliably achieved. Here, "particle size" refers to a 50% cumulative particle size by volume measured by a laser diffraction particle size distribution analyzer, and the particle size hereinafter is measured in the same manner.

### (Coating Layer)

The coating layer is provided on the surface of the magnetic metal particle and is constituted from silicon oxide. The coating layer covers the magnetic metal particle and acts to suppress elution of metal components from the magnetic metal particle when forming the polymer layer.

The thickness of the coating layer is not particularly limited; however, if it is excessively thin, the surface of the magnetic metal particle may not be uniformly covered. Therefore, from the viewpoint of more reliably suppressing elution of components from the magnetic metal particle when forming the polymer layer, the thickness of the coating layer is preferably 1 nm or more. On the other hand, if the coating layer is excessively thick, the magnetic properties of the test particle, such as saturation magnetization and coercivity, may decrease. Therefore, from the viewpoint of maintaining high magnetic properties, the thickness of the coating layer is preferably 80 nm or less. The thickness of the coating layer can be measured, for example, by observing a cross section of the coating layer using a transmission electron microscope (TEM) or a scanning electron microscope (SEM) and determining the average film thickness. Specifically, TEM or SEM images of the cross section are taken, and the average film thickness can be obtained from the average of 50 measurement points on any particle.

### (Polymer Layer)

The polymer layer is provided on the surface of the coating layer. The polymer layer includes a polymer of a structural unit represented by formula (1) and alkoxysilane having an acrylic group or a methacrylic group, and is configured to bind with carrier substances that can capture the target substance. Here, the target substance refers, for example, to proteins, nucleic acids, cells, and the like contained in the blood that are to be tested. The carrier substances are not particularly limited as long as it can capture the target substance and can be appropriately selected according to the type of target substance. Examples of the carrier substances include streptavidin, protein A, protein G, and antibodies. 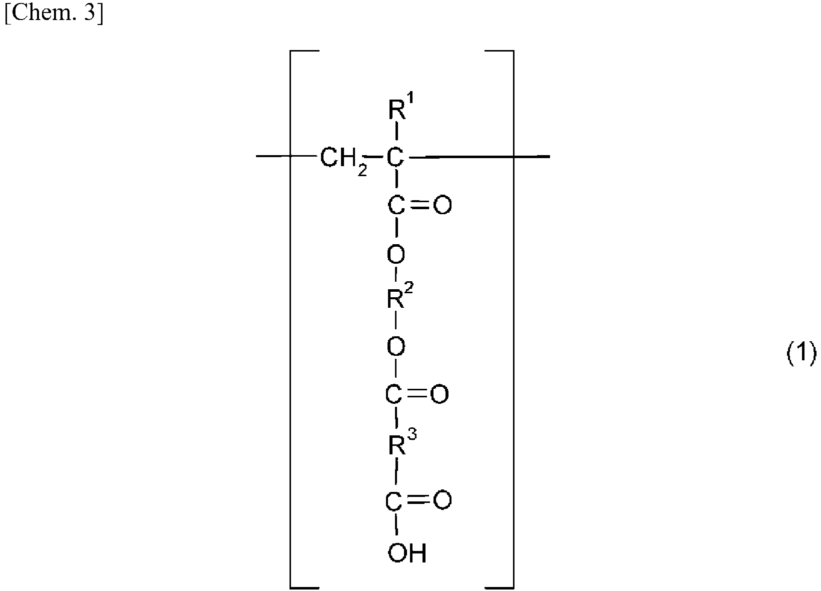 In formula (1), R¹ represents a hydrogen atom or a methyl group, R² represents a linear or branched alkylene group having 2 or more and 6 or less carbon atoms, and R³ represents an alkylene group having 2 or more and 6 or less carbon atoms, a cyclohexylene group or a phenylene group.

The polymer has a carboxyl group (-COOH group) at the terminal of the structural unit of formula (1), which enables binding to the carrier substances. The polymer includes R² and R³ as shown in the chemical structure of formula (1), and the molecular chain thereof is long, resulting in a bulky molecule. Therefore, the polymer can easily bind to carrier substances with large molecular sizes, such as streptavidin. That is, the polymer layer allows increasing the amount of carrier substances attached to the surface thereof (carried amount). Regarding the alkylene groups of R² and R³, when the number of carbon atoms is 1, the molecular chain becomes short and the carried amount may decrease, which is undesirable. When the number of carbon atoms is 7 or more, hydrophobicity increases, making the polymerization reaction during the polymer layer forming step difficult and potentially reducing productivity, which is also undesirable.

In the structural unit of formula (1), it is preferable that R² is an ethylene group and R³ is an alkylene group or a phenylene group, and it is more preferable that R³ is a phenylene group. Such a structural unit allows stable formation of the polymer while reliably increasing the carried amount of the carrier substances.

As a silane coupling agent for forming the polymer, any alkoxysilane having an acrylic group or a methacrylic group that can polymerize with the compound of formula (2) may be used without particular limitation. From the viewpoint of more reliably increasing the carried amount of carrier substances in the polymer layer, it is preferable that the silane coupling agent has a long molecular chain and a bulky chemical structure. Specifically, it is preferable that the silane coupling agent is an alkoxysilane having: an acrylic group or a methacrylic group; and an alkylene group having 3-6 carbon atoms. Further, to increase reactivity with the compound of formula (2) and ensure reliable polymerization of the polymer, it is preferable that the silane coupling agent has an acryloxypropyl group or a methacryloxypropyl group.

### (Carbon Content)

The amount of polymer contained in the test particle can be simply quantitatively evaluated as carbon content. From the viewpoint of ensuring high magnetic properties, the carbon content of the test particle is preferably 0.1-10 mass %, and may be 0.5-10 mass %. If the carbon content is excessively low, the polymer layer cannot uniformly cover the surface of the coating layer, and the carried amount of carrier substances may not be maintained at high level. Conversely, if the carbon content is excessively high, the proportion of magnetic components in the test particle decreases, making it difficult to maintain high magnetic properties. With the carbon content in the above range, a good balance between the carried amount of carrier substances and magnetic properties can be achieved at a high level. Here, carbon content refers to the amount of carbon derived from the polymer layer relative to 100 parts by mass of the test particle (polymer-coated magnetic metal particle). Carbon content can be measured, for example, using a carbon/sulfur analyzer as described in the Examples.

### (Particle Size)

The particle size of the test particle is not particularly limited, but preferably 0.2-10 µm. As the particle size decreases, the volume of the magnetic particle also decreases, making it more difficult to achieve magnetic responsiveness when magnetism is applied, which is undesirable. If the particle size is too large, the test particle tends to settle in the solution, which is also undesirable.

### (Properties)

The test particle of the present embodiment is configured such that a coating layer and a polymer layer are sequentially laminated on the surface of the core magnetic metal particle. Accordingly, the test particle exhibits the following properties.

In the test particle, the polymer layer includes a polymer of a structural unit represented by formula (1) and alkoxysilane having an acrylic group or a methacrylic group, and can easily bind with carrier substances that capture the target substance. That is, the test particle is configured to carry a larger amount of carrier substances. Specifically, streptavidin is carried on the test particle as the carrier substances, and ALP-biotin (alkaline phosphatase-biotin) and a luminescent dye (e.g., Lumi-Phos Plus) are bound to 50 µg of this streptavidin-immobilized particle, and the luminescence intensity is measured. A calibration curve showing the correlation between luminescence intensity and ALP-biotin concentration is created, and the ALP-biotin concentration is calculated from the luminescence intensity, which is regarded as the biotin binding amount. The test particle of the present embodiment has a high biotin binding amount and excellent carrier-substances-carrying capacity.

Further, since the core of the test particle is a magnetic metal particle, the proportion of magnetic components in the particle is higher than that of a magnetic polymer particle in which the core is a polymer particle and magnetic particles are attached around it. Accordingly, the test particle has high saturation magnetization. Specifically, it is preferable that the saturation magnetization of the test particle is 100 Am²/kg or more. The upper limit is not particularly limited, but for example, it may be 210 Am²/kg or less. With a test particle having such saturation magnetization, magnetic responsiveness is high, allowing reduction of the time required to recover the test particle under a magnetic field. The method for measuring saturation magnetization is described in detail in the Examples.

Moreover, since the core of the test particle is a magnetic metal particle, the test particle is configured to have lower coercivity than a magnetic polymer particle. Specifically, it is preferable that the coercivity Hc of the test particle is 20 Oe or less. The lower limit is not particularly limited, but for example, it may be 3 Oe or more. A test particle having such coercivity can enhance particle dispersibility.

### (2) A Method of Producing a Polymer-Coated Magnetic Metal Particle

Next, a method of producing a polymer-coated magnetic metal particle is described. The method of the present embodiment includes a preparation step, a coating layer forming step, and a polymer layer forming step. Each step is described in detail below.

### (Preparation Step)

First, the core magnetic metal particle is prepared. It is preferable that the saturation magnetization of the magnetic metal particle to be prepared is 100Am²/kg or more and 210 Am²/kg or less. Moreover, from the viewpoint of ensuring excellent saturation magnetization of the resulting test particle, it is preferable to use an iron particle or an iron-based alloy particle as the magnetic metal particle.

### (Coating Layer Forming Step)

Next, a coating layer constituted from silicon oxide is formed on the surface of the magnetic metal particle. As the method for forming the coating layer, a sol-gel method can be employed, for example.

Specifically, first, the magnetic metal particle is added to a solvent containing water to obtain a slurry in which the magnetic metal particle is dispersed. Next, while stirring the slurry, a silicon alkoxide is added thereto. The alkoxy groups of the silicon alkoxide hydrolyze in the presence of water to produce silanol derivatives. The addition amount of silicon alkoxide is preferably 0.1-5.0 parts by mass of Si based on 100 parts by mass of the magnetic metal particle. The silanol derivatives adhere to the surface of the magnetic metal particle and form a reaction layer. After a predetermined time following the addition of the silicon alkoxide, a hydrolysis catalyst is added while stirring the solvent containing the slurry. This hydrolyzes any remaining alkoxy groups in the silanol derivatives. Simultaneously with the addition of the hydrolysis catalyst, the solvent is heated. The heating causes the silanol derivatives to condense or polymerize, forming a polysiloxane structure, and further heating results in the formation of silica (SiO₂). Finally, the solvent is dried to obtain a silica-coated particle in which a coating layer constituted from silicon oxide is formed on the surface of the magnetic metal particle.

As the silicon alkoxide, conventionally known compounds can be used, for example, trimethoxysilane, tetramethoxysilane, triethoxysilane, tetraethoxysilane, tripropoxysilane, tetrapropoxysilane, tributoxysilane, and the like.

As the hydrolysis catalyst, it is preferable to use an alkaline catalyst from the viewpoint of suppressing dissolution of the metal component constituting the magnetic metal particle. As the alkaline catalyst, for example, ammonia water can be used.

The addition amounts of the silicon alkoxide and the hydrolysis catalyst, as well as the reaction times of the silicon alkoxide and the hydrolysis catalyst may be appropriately changed depending on the thickness of the coating layer, and, for example, may be appropriately adjusted so that the coating layer thickness is 1-80 nm. Further, from the viewpoint of the reactivity of hydrolysis of the silicon alkoxide and the adhesion of silanol derivatives to the surface of the magnetic metal particle, the heating temperature of the solvent containing the slurry may be, for example, 20-70 °C. As the solvent, either water alone or a mixed solvent containing water and an organic solvent can be used.

### (Polymer Layer Forming Step)

Next, a polymer layer is formed on the surface of the silica-coated particle.

Specifically, first, the silica-coated particle and a solvent containing water are mixed to prepare a dispersion liquid. Next, while stirring the dispersion liquid, an alkoxysilane having an acrylic group or a methacrylic group is added, as a silane coupling agent, and mixed. The alkoxy groups of the alkoxysilane are considered to hydrolyze by reaction with water and bind to the coating layer constituted from silicon oxide. After adding the silane coupling agent, preferably, an alkali such as NaOH or NH₃ is added to adjust the pH of the solution to 8 or more, followed by addition of a compound represented by formula (2). The addition of the alkali has the effect of improving the reactivity of the silane coupling agent and the solubility of the compound of formula (2) in water. It is considered that, starting from the silane coupling agent bound to the coating layer, the acrylic group or methacrylic group of the silane coupling agent binds with the acrylic group or methacrylic group in the chemical structure of the compound of formula (2), or with the acrylic group or methacrylic group of unbound silane coupling agent, thereby forming the polymer layer. The polymer constituting the polymer layer has the structural unit shown in formula (1) and the chemical structure derived from the silane coupling agent. 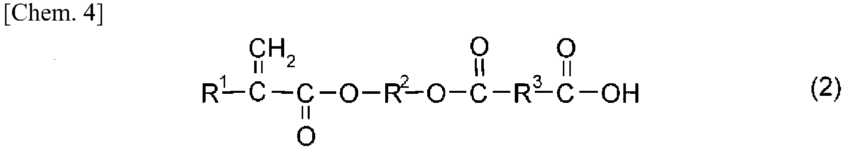 In formula (2), R¹ represents a hydrogen atom or a methyl group, R² represents a linear or branched alkylene group having 2 or more and 6 or less carbon atoms, and R³ represents an alkylene group having 2 or more and 6 or less carbon atoms, a cyclohexylene group or a phenylene group. For the alkylene groups of R² and R³, when the number of carbons is 1, the molecular chain becomes short, which is undesirable because the carried amount of carrier substances may decrease. Conversely, when the number of carbons is 7 or more, hydrophobicity increases, which may make the polymerization reaction more difficult and may reduce productivity, which is also undesirable.

As the compound of formula (2), from the viewpoint of reactivity with the silane coupling agent and improving binding between a polymer layer and a carrier substance to increase the carried amount, it is preferable that R² is an ethylene group, and R³ is an alkylene group or a phenylene group. From the viewpoint of further increasing the amount of carrier substances carried on the polymer layer, it is more preferable that R³ is a phenylene group, as shown in the Examples described later.

As the silane coupling agent, an alkoxysilane having an acrylic group or a methacrylic group that can polymerize with the compound of formula (2) is used. From the viewpoint of more reliably increasing the amount of carrier substances carried on the polymer layer, it is preferable that this alkoxysilane has a long molecular chain and a bulky chemical structure, specifically, has: an acrylic group or a methacrylic group; and an alkylene group having 3-6 carbon atoms. Moreover, from the viewpoint of increasing reactivity with the compound of formula (2) and ensuring more reliable polymerization of the polymer, it is preferable that this alkoxysilane has an acryloxypropyl group or a methacryloxypropyl group.

The addition amounts of the silane coupling agent and the compound of formula (2) may be adjusted within a range that allows formation of the polymer layer at a desired thickness. For example, the addition amount of the silane coupling agent is preferably 0.1-40 parts by mass relative to 100 parts by mass of the magnetic metal particle on which the coating layer is formed, more preferably 5.0-40 parts by mass, and even more preferably 15-40 parts by mass. It may also be 0.1-10 parts by mass. By setting the addition amount of the silane coupling agent to 15-40 parts by mass relative to 100 parts by mass of the magnetic metal particle on which the coating layer is formed, the storage stability of the magnetic metal particle on which the coating layer is formed can be improved. The addition amount of the compound of formula (2) is preferably 100-700 parts by mass relative to 100 parts by mass of the magnetic metal particle on which the coating layer is formed, and may be 100-500 parts by mass. Moreover, the addition amount of the compound of formula (2) is preferably 10-100 times by mass relative to the addition amount of the silane coupling agent. Here, "storage stability" refers to the stability that allows the carrier substances carried on the polymer-coated magnetic metal particle to remain carried even after the passage of time. Specifically, the retention ratio α of the carried amount, which is an index of storage stability, is calculated as α = B/A × 100 [%], where A is the initial amount of carrier substances carried on the polymer-coated magnetic metal particle, and B is the carried amount after a predetermined time. High storage stability indicates that the carried amount B after the predetermined time does not vary significantly from the initial carried amount A. The retention ratio α is not particularly limited, but it is preferably 30% or more.

In forming the polymer, it is preferable to add a polymerization initiator after adding the compound of formula (2). By adding the polymerization initiator, binding between the silane coupling agent and the compound of formula (2) can be further promoted. From the viewpoint of reactivity, a water-soluble azo polymerization initiator having a carboxyl group is preferably used as the polymerization initiator. The addition amount of the polymerization initiator is preferably 10-100 parts by mass relative to 100 parts by mass of the magnetic metal particle on which the coating layer is formed.

After completion of the polymerization reaction, the test particle, in which the polymer layer has been formed on the surface of the coating layer, is recovered from the solvent using, for example, a magnet, and then washed. In this way, the test particle of the present embodiment is obtained.

A method for measuring a target substance using the above test particle is, for example, as follows. First, the carrier substances are bound to the test particle to obtain carrier substances-immobilized particles. This carrier substances-immobilized particle is added to a sample solution. Next, the carrier substances-immobilized particle captures the target substance contained in the sample solution. Subsequently, the carrier substances-immobilized particles dispersed in the sample solution are recovered with a magnetic field. Then, the target substance captured by the recovered carrier substances-immobilized particles is measured using a conventionally known method.

Although embodiments of the present disclosure have been described above in detail, the present disclosure is not limited to the above embodiments, and various modifications may be made without departing from the gist of the disclosure.

### Examples

Next, the present disclosure will be described in more detail with reference to Examples; however, the present disclosure is not limited to these Examples. In this Example, a coating layer and a polymer layer were formed on the surface of a magnetic metal particle to produce a polymer-coated magnetic metal particle.

### <Example 1> (1) Production of Polymer-Coated Magnetic Metal Particle

First, carbonyl iron powder (BASF, HS grade, D50: 2.3 µm) was prepared as the magnetic metal particle.

Next, a coating layer constituted from silicon oxide was formed on the surface of the carbonyl iron powder. Specifically, 5,451 g of isopropyl alcohol and 820 g of pure water were added to a 10 L reaction tank and stirred under a nitrogen atmosphere. To this solution, 1,650 g of carbonyl iron powder (hereinafter also referred to as Fe particles) was added and stirred at 40 °C. Subsequently, 257.4 g of tetraethoxysilane (Wako Pure Chemical Industries) and 50 g of isopropyl alcohol were added and stirred for 5 minutes. Thereafter, 567.3 g of 25 mass% ammonia water was added over 90 minutes. At the start of the addition of ammonia water, a feed pump was operated to feed the solution to a high-pressure homogenizer (LAB1000, SMT Co., Ltd.). Simultaneously with the fluid feed, the high-pressure homogenizer was set to a pressure of 150 bar to perform dispersion treatment. The reaction solution after the dispersion treatment was set to return to the 10 L reaction tank. After the addition of ammonia water, the mixture was stirred for 60 minutes. Dispersion treatment was continued until the reaction was complete. The resulting slurry was filtered, and the cake was dried at 110 °C under a nitrogen atmosphere. In this manner, silica coated particles with a silicon oxide coating layer formed on the surface of the carbonyl iron powder were obtained.

Next, a polymer layer was formed on the surface of the silica coated particle. Specifically, 1.88 g of silica coated particles (silica content 0.04 g) and 30 g of pure water were mixed and subjected to sonication for 30 minutes. The sonicated suspension and 204.76 g of pure water were added to a 300 mL separable beaker and bubbled with nitrogen gas at 0.1 L/min for 30 minutes. The nitrogen gas flow path was switched from bubbling into the liquid to flowing into the space above the liquid, and the mixture was stirred while heating to 35 °C. After heating, 0.119 g of 3-methacryloxypropyltrimethoxysilane (Shin-Etsu Chemical Co., Ltd.), shown in formula (3), was added as a silane coupling agent and stirred for 30 minutes. Next, 6.67 g of 2-methacryloyloxyethyl phthalate (Tokyo Chemical Industry Co., Ltd.), shown in formula (4), as a compound (monomer) of formula (2), and 5 mL of 5 wt% sodium hydroxide aqueous solution were added and stirred for 30 minutes. Subsequently, the mixture was stirred for an additional 30 minutes while heating to 65 °C. Then, 0.994 g of 2,2'-azobis[N-(2-carboxyethyl)-2-methylpropionamidine] n-hydrate (Wako Pure Chemical Industries) was dissolved in 5 g of pure water and added to the reaction tank. After addition, the mixture was stirred for 4 hours. After completion of the polymerization reaction, the particles were recovered with a magnet, and the supernatant was removed. The particles were then redispersed in 50 g of pure water and recovered with a magnet, and the supernatant was removed. This washing operation from redispersion in pure water to removal of the supernatant was repeated four more times, and the resulting particles were dispersed in pure water. In this manner, a slurry in which the polymer-coated magnetic metal particles of Example 1 were dispersed was obtained.

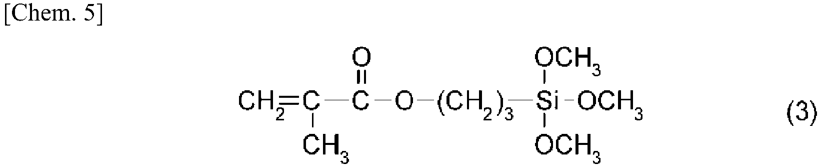

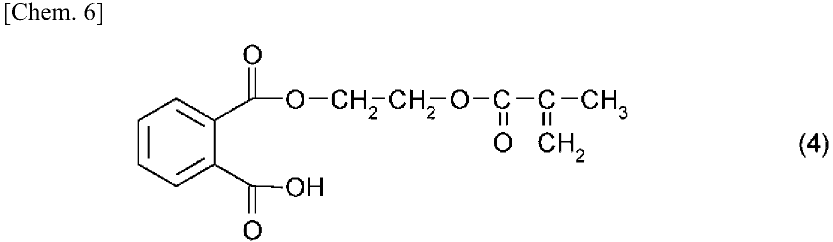

The production conditions of Example 1 are shown in Table 1 below.

**[Table 1]**

| | Material | | | |
|---|---|---|---|---|
| | Magnetic metal particle | Silane coupling agent (alkoxysilane) | Amount of coupling agent added per 1g of magnetic particles (g) | Compound of formula (2) (monomer) |
| Example 1 | Fe particle | 3-Methacryloxypropyltrimethoxysilane | 0.063 | 2-Methacryloyloxyethyl phthalate |
| Example 2 | | | 0.063 | 2-Acryloyloxyethyl succinate |
| Example 3 | Fe-B alloy particle | | 0.063 | 2-Methacryloyloxyethyl phthalate |
| Example 4 | Fe particle | 3-Acryloxypropyltrimethoxysilane | 0.063 | |
| Example 5 | Fe-Ni alloy particle | 3-Methacryloxypropyltrimethoxysilane | 0.063 | 2-Methacryloyloxyethyl phthalate |
| Com.Ex. 1 | Fe particle | 3-Methacryloxypropyltrimethoxysilane | 0.063 | Styrene |
| Com.Ex. 2 | | | 0.063 | 2-Carboxyethyl acrylate |
| Com.Ex. 3 | | | 0.063 | 4-Vinylbenzoic acid |
| Com.Ex. 4 | | Trimethoxy-4-vinylphenylsilane | 0.063 | 2-Methacryloyloxyethyl phthalate |
| Com.Ex. 5 | | Triethoxyvinylsilane | 0.063 | |

| | | | | |
|---|---|---|---|---|
| *Com.Ex. = Comparative Example | | | | |

### (2) Evaluation

The produced polymer-coated magnetic metal particles were evaluated for the carried amount of carrier substances, magnetic properties such as saturation magnetization and coercivity, carbon content, and particle size by the following methods.

### (Carried amount)

The carried amount of carrier substances was evaluated based on the biotin binding amount per unit mass of the test particle. In the following, EDC refers to 1-Ethyl-3-(3-dimethylaminopropyl)carbodiimide Hydrochloride (Tokyo Chemical Industry Co., Ltd.), MES refers to 2-(N-morpholino)ethanesulfonic acid, NHS refers to N-Hydroxysuccinimide (Wako Pure Chemical Industries), PBS refers to phosphate-buffered saline, TBS refers to tris-buffered saline, and TBST refers to TBS containing Tween 20. First, the slurry in which polymer-coated magnetic metal particles were dispersed was dried by heating to obtain polymer-coated magnetic metal particles. To 1 mg of the obtained polymer-coated magnetic metal particles, 1 mL of 0.01 mol/L MES buffer solution containing EDC at 5 mg/mL was added and stirred for 30 minutes using a vortex mixer. After stirring, the magnetic particles were separated with a magnet and washed three times with 1 mL of 0.01 mol/L MES buffer solution. Next, 1 mL of 0.01 mol/L MES buffer solution containing NHS at 0.8 mg/mL was added and stirred for 30 minutes using a vortex mixer. After stirring, the magnetic particles were separated with a magnet and washed three times with 1 mL of 0.01 mol/L MES buffer solution. Subsequently, 1 mL of 0.01 mol/L PBS buffer solution (pH 5.7) containing streptavidin (Wako Pure Chemical Industries) at 0.2 mg/mL was added and stirred for 30 minutes using a vortex mixer. After stirring, the magnetic particles were separated with a magnet and washed three times with 1 mL of 0.01 mol/L PBS buffer solution to obtain streptavidin-immobilized particles. Then, 100 µL of 1 × TBS buffer solution containing ALP-biotin (Thermo Fisher Scientific) at 0.01 mg/mL was added to 50 µg of the streptavidin-immobilized particles and let it stand for 30 minutes. After standing, the particles were separated with a magnet and washed three times with 200 µL of TBST buffer solution. The particles were then suspended in 50 µL of TBS, transferred to a well plate, and 50 µL of the luminescent dye Lumi-Phos Plus (Wako Pure Chemical Industries) was added. After 5 minutes, the luminescence intensity was measured using a microplate reader. A calibration curve was created from the luminescence intensity versus ALP-biotin concentration, and the biotin binding amount was calculated from this calibration curve. The biotin binding amount per unit mass of the test particle of Comparative Example 1 was set to 100, and the biotin binding amount of the present Example was calculated as a relative value. When the relative value is 130 or higher, it is evaluated that the biotin binding amount is high and the amount of the carrier substances carried is high. For the measurement of luminescence intensity, a microplate reader (SH-9000Lab, Corona Electric Co., Ltd.) was used, with the measurement conditions set to luminescence and a gate time of 1.0 s.

### (Storage Stability)

The storage stability was evaluated using the streptavidin-immobilized particles obtained by the above carried amount evaluation method. In this Example, the initial biotin binding amount and the biotin binding amount after a predetermined time regarding the streptavidin-immobilized particles obtained by the above carried amount evaluation method were measured, and the retention rate was calculated. Specifically, for the streptavidin-immobilized particles obtained in the carried amount evaluation, the calculated biotin binding amount was taken as the initial biotin binding amount. Meanwhile, the streptavidin-immobilized particles were left stand at 37 °C for 4 days. After the storage period, 50 µg of the streptavidin-immobilized particles was mixed with 100 µL of 1 × TBS buffer solution containing ALP-biotin (Thermo Fisher Scientific) (0.01 mg/mL) and left stand for 30 minutes. The particles were then separated with a magnet and washed three times with 200 µL of TBST buffer solution. The particles were suspended in 50 µL of TBS, transferred to a well plate, and 50 µL of Lumi-Phos Plus (Wako Pure Chemical Industries) was added. After 5 minutes, the luminescence intensity was measured using a microplate reader. The calibration curve of luminescence intensity versus ALP-biotin concentration was created and used to calculate the biotin binding amount, which was taken as the biotin binding amount after 4 days at 37 °C. The storage stability was evaluated by the retention rate calculated by the following formula. In this Example, a retention rate of 30% or more was considered to indicate excellent storage stability. 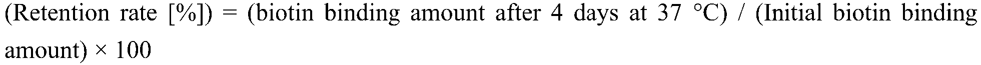 (Retention rate [%]) = (biotin binding amount after 4 days at 37 °C)/(Initial biotin binding amount) × 100

### (Magnetic properties)

The slurry in which polymer-coated magnetic metal particles were dispersed was dried by heating to obtain polymer-coated magnetic metal particles. The obtained polymer-coated magnetic metal particles were measured for saturation magnetization and coercivity using a vibrating sample magnetometer (VSM) (VSM-5, Toei Kogyo Co., Ltd.) with an applied magnetic field of 798 kA/m (10 kOe), an M measurement range of 0.05 Am² (50 emu), a time constant of 0.03 s, and a wait time of 0.1 s. The accompanying software (Ver. 2.1, Toei Kogyo Co., Ltd.) was used for the measurements. In this Example, when the saturation magnetization was 100 Am²/kg or more, the polymer-coated magnetic metal particles could be rapidly recovered under a magnetic field, indicating excellent magnetic responsiveness. When the coercivity was 20 Oe or less, the polymer-coated magnetic metal particles dispersed quickly after removing the magnetic field, indicating excellent dispersibility. In Table 1, the coercivity Hc is shown in both [Oe] and [kA/m].

### (Carbon content)

The slurry in which polymer-coated magnetic metal particles were dispersed was dried by heating to obtain polymer-coated magnetic metal particles. The polymer-coated magnetic metal particles obtained by heating and drying the slurry were measured for carbon content using a carbon/sulfur analyzer (EMIA-920V2, HORIBA, Ltd.).

### (Particle Size)

The particle size of the polymer-coated magnetic metal particles was measured based on the particle size distribution of the slurry in which polymer-coated magnetic metal particles were dispersed using a laser diffraction/scattering particle size distribution analyzer (SYNC, Microtrac Bell Co., Ltd.). From the obtained volume-based particle size distribution, the 50% cumulative particle size D50 (unit: µm) was determined.

### (3) Evaluation Results

The results of each evaluation are summarized in Table 2.

**[Table 2]**

| | Evaluation | | | | | | |
|---|---|---|---|---|---|---|---|
| | D50 [µm] | Biotin binding amount | Saturation magnetization σs [Am²/kg] | Coercivity Hc | | Carbon content [%] | Storage stability retention rate (%) |
| | | | | [Oe] | [kA/m] | | |
| Example 1 | 2.2 | 303 | 197 | 5 | 0.4 | 3 | 37 |
| Example 2 | 2.9 | 159 | 183 | 6 | 0.5 | 4 | - |
| Example 3 | 0.4 | 285 | 109 | 5 | 0.4 | 6 | - |
| Example 4 | 2.6 | 216 | 140 | 6 | 0.5 | 8 | - |
| Example 5 | 0.9 | 183 | 143 | 19 | 1.5 | 5 | 32 |
| Com.Ex. 1 | 2.6 | 100 | 182 | 6 | 0.5 | 3 | - |
| Com.Ex. 2 | 2.5 | 72 | 190 | 6 | 0.5 | 1 | - |
| Com.Ex. 3 | 2.3 | 50 | 196 | 5 | 0.4 | 1 | - |
| Com.Ex. 4 | 2.5 | 120 | 94 | 9 | 0.7 | 36 | - |
| Com.Ex. 5 | 2.4 | 115 | 86 | 10 | 0.8 | 47 | - |

| | | | | | | | |
|---|---|---|---|---|---|---|---|
| *Com.Ex. = Comparative Example | | | | | | | |

As shown in Table 2, the polymer-coated magnetic metal particles of Example 1 had a D50 of 2.2 µm, a biotin binding amount of 303, a saturation magnetization of 197 Am²/kg, a coercivity Hc of 5 Oe, and a carbon content of 3%. That is, in Example 1, it was confirmed that the carried amount of the carrier substances was high, the saturation magnetization was high indicating excellent magnetic responsiveness, and the coercivity was low indicating excellent magnetic separability. Observation of the coating layer thickness by a transmission electron microscopy confirmed that the thickness ranged from 1-80 nm.

The high carried amount was achieved because the polymer layer was formed using an alkoxysilane having an acrylic group or a methacrylic group and the compound of formula (2), 2-methacryloyloxyethyl phthalate. In Example 1, the polymer forming the polymer layer contains structural units derived from 2-methacryloyloxyethyl phthalate, which satisfy formula (1), and structural units derived from the predetermined alkoxysilane. The structural units derived from 2-methacryloyloxyethyl phthalate include ethyl groups, methacrylic groups, and phenylene groups, resulting in long molecular chains. Therefore, the polymer layer can readily bind to streptavidin and increase the carried amount. In Example 1, since the core was an Fe particle, the saturation magnetization could be increased to enhance magnetic responsiveness while the coercivity could be kept low to improve magnetic separability. Furthermore, with the carbon content derived from the polymer layer in the range of 0.5-10 mass% and the polymer layer formed at an appropriate thickness, both carried amount and magnetic properties could be balanced.

In addition, the polymer-coated magnetic metal particles of Example 1 exhibited a high retention rate of 37% for the carried amount, indicating excellent storage stability. This is considered to be because the silane coupling agent was added at 5.0-40 parts by mass per 100 parts by mass of the magnetic metal particles on which the coating layer was formed, and the compound of formula (2) was added at 100-500 parts by mass per 100 parts by mass of the magnetic metal particles on which the coating layer was formed, allowing the polymer layer to be uniformly formed on the coating layer.

### (Examples 2-5)

In Examples 2-5, polymer-coated magnetic metal particles were produced in the same manner as in Example 1, except that the types of alkoxysilane, the compound of formula (2), and the magnetic metal particles were appropriately changed as shown in Table 1.

As the alkoxysilane, 3-acryloxypropyltrimethoxysilane shown in formula (5) was used.

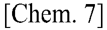

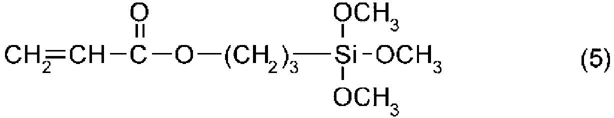

As the compound of formula (2), 2-acryloyloxyethyl succinate shown in formula (6) was used.

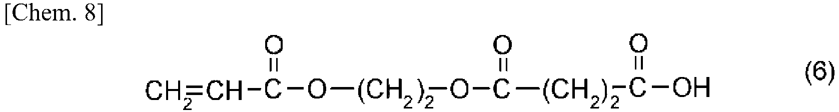

As the magnetic metal particles, Fe-B alloy particles or Fe-Ni alloy particles was used. The Fe-B alloy particles were produced as follows. First, 11.3 g of ferrous chloride (FeCl₂), 15.9 g of ammonium chloride, and 62.3 g of sodium gluconate were dissolved in 285.7 g of water at 30°C in a 500 mL beaker. Then, 28.2 g of 25% ammonia water was added to adjust the pH of the solution to 9. The solution was heated to 50 °C and stirred at 300 rpm while a solution prepared by mixing 21.7 g of sodium borohydride in 214.2 g of water was added, followed by maturation for 10 minutes. The resulting particles were recovered with a magnet and washed with ethanol to obtain Fe-B alloy particles. The boron content was 9.2 wt%. The Fe-Ni alloy particles used had an Fe content of 50% and a Ni content of 50%.

The polymer-coated magnetic metal particles of Examples 2-5 were evaluated in the same manner as in Example 1. As shown in Table 2, Examples 2-5 exhibited similarly high biotin binding amounts, high saturation magnetization, and low coercivity, comparable to Example 1. Furthermore, in Examples 2-5, the coating layer thickness and storage stability were confirmed to be approximately the same as in Example 1.

In Examples 1-5, it was confirmed that, since an alkoxysilane having an acrylic group or a methacrylic group and an alkylene group having 3-6 carbon atoms, specifically, an alkoxysilane having an acryloxypropyl or methacryloxypropyl group was used as the silane coupling agent, the carried amount on the polymer-coated magnetic metal particles could be increased.

When comparing Examples 1-5, Examples 1, 3, and 4, which used a phthalate in formula (4) with R³ as a phenylene group, exhibited higher biotin binding amounts than Examples 2 and 5, which used a succinate in formula (6) with R³ as an alkylene group. This suggests that introducing a bulkier chemical structure in the polymer layer increases the streptavidin carrying amount, thereby improving the capacity to capture biotin.

### (Comparative Examples 1-5)

In Comparative Examples 1-5, polymer-coated magnetic metal particles were produced in the same manner as in Example 1, except that the type of alkoxysilane was changed to the compounds shown in formulas (7), (8) and the type of compound of formula (2) was changed to the compounds shown in formulas (9)-(11), as shown in Table 1. Formula (7) represents trimethoxy-4-vinylphenylsilane, formula (8) represents triethoxyvinylsilane, formula (9) represents styrene, formula (10) represents 2-carboxyethyl acrylate, and formula (11) represents 4-vinylbenzoic acid. The polymer-coated magnetic metal particles of Comparative Examples 1-5 were evaluated in the same manner as in Example 1. The results are shown in Table 2.

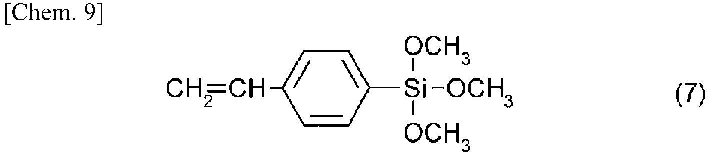

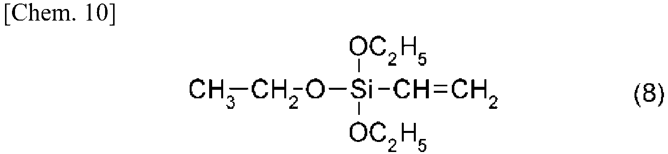

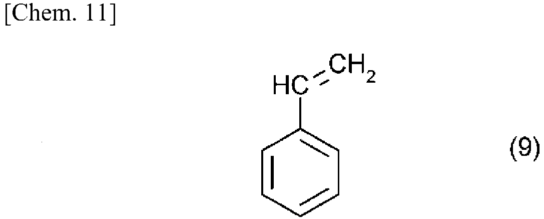

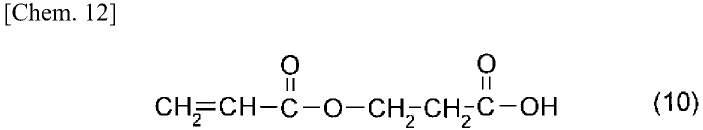

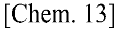

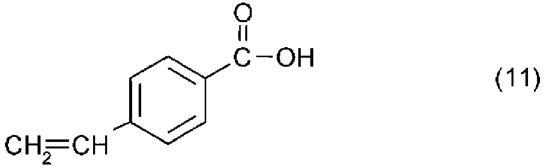

As shown in Table 2, in Comparative Examples 1-3, although the saturation magnetization was high and the coercivity was low, the biotin binding amount was lower compared to Examples 1-5. This is because, in forming the polymer layer, compounds satisfying formula (2) were not used; instead, styrene, 2-carboxyethyl acrylate, and 4-vinylbenzoic acid were employed. In Comparative Examples 1-3, unlike Examples 1-5, the polymer layer could not incorporate long molecular chains or a bulky chemical structure, resulting in inefficient binding with streptavidin.

Furthermore, in Comparative Examples 4 and 5, as shown in Table 2, both the biotin binding amount and the magnetic properties tended to be lower compared to Examples 1-5. This is because, in forming the polymer layer, the silane coupling agent used was not an alkoxysilane having an acrylic group or a methacrylic group; instead, trimethoxy-4-vinylphenylsilane or triethoxyvinylsilane, which lack acrylic group or a methacrylic groups, were used. In Comparative Examples 4 and 5, the polymer layer could not be structured to readily bind to streptavidin, nor could the carbon content be adjusted to 0.5-10 mass%. Since the biotin binding amounts in Comparative Examples 1-5 were low, storage stability was not evaluated.

### (Examples 6-16)

In Examples 6-16, as shown in Table 3, polymer-coated magnetic metal particles were produced in the same manner as in Example 1, except that the types of alkoxysilane (silane coupling agent), the compound of formula (2), and the magnetic metal particles were appropriately changed. The produced polymer-coated magnetic metal particles were evaluated in the same manner as in Example 1. The evaluation results for Examples 6-16 are summarized in Table 4.

As the alkoxysilane, either 3-methacryloxypropylmethyldimethoxysilane shown in formula (12) or 3-methacryloxypropylmethyldiethoxysilane shown in formula (13) was used.

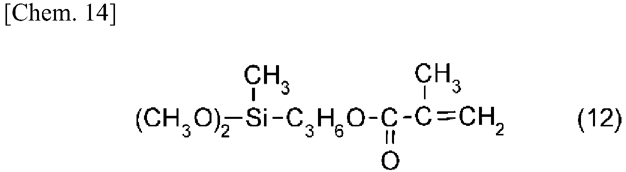

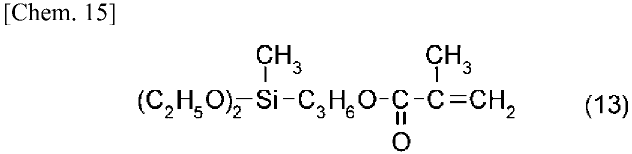

As the compound of formula (2), the following were used: 2-methacryloyloxyethyl succinate shown in formula (14), 2-acryloyloxyethyl phthalate shown in formula (15), and 2-acryloyloxyethyl hexahydrophthalate shown in formula (16).

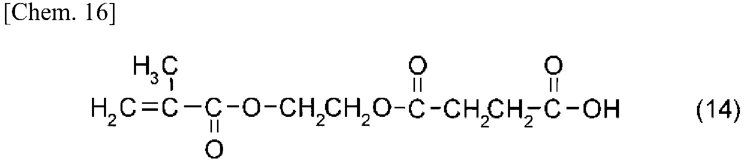

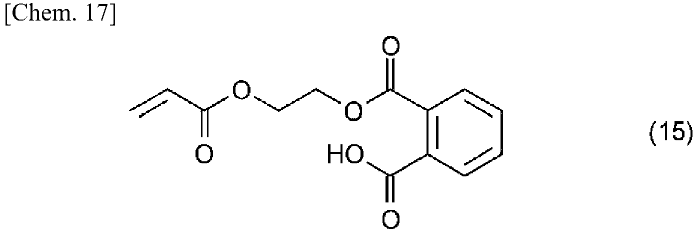

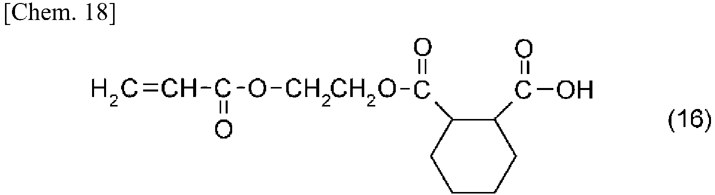

**[Table 3]**

| | Material | | | |
|---|---|---|---|---|
| | Magnetic metal particle | Silane coupling agent (alkoxysilane) | Amount of coupling agent added per 1g of | Compound of formula (2) (monomer) |
| | | | magnetic particles (g) | |
| Example 6 | Fe-Ni alloy particle | | 0.20 | 2-Methacryloyloxyethyl phthalate |
| Example 7 | | | 0.20 | 2-Acryloyloxyethyl succinate |
| Example 8 | | | 0.20 | 2-Methacryloyloxyethyl succinate |
| Example 9 | | | 0.20 | 2-Acryloyloxyethyl phthalate |
| Example 10 | Fe particle | 3-Methacryloxypropylmethyldimethoxysilane | 0.23 | 2-Methacryloyloxyethyl phthalate |
| Example 11 | | | 0.23 | 2-Acryloyloxyethyl succinate |
| Example 12 | | | 0.23 | 2-Methacryloyloxyethyl succinate |
| Example 13 | | | 0.23 | 2-Acryloyloxyethyl phthalate |
| Example 14 | | | 0.23 | 2-Acryloyloxyethyl hexahydrophthalate |
| Example 15 | | 3-Methacryloxypropylmethyldiethoxysilane | 0.26 | 2-Methacryloyloxyethyl phthalate |
| Example 16 | | 3-Acryloxypropyltrimethoxysilane | 0.23 | 2-Methacryloyloxyethyl phthalate |

In Example 6, similarly to Example 5, a silicon oxide coating layer was formed on the surface of Fe-Ni alloy particles having an Fe content of 50% and a Ni content of 50% to obtain silica-coated particles. Subsequently, a polymer layer was formed on the surface of the silica-coated particles. Specifically, 8.00 g of silica-coated particles (silica content 0.16 g) was mixed with 40 g of pure water and sonicated for 10 minutes. The sonicated suspension and 488.63 g of pure water were placed in a 1 L beaker, and bubbled with nitrogen gas at 0.1 L/min for 30 minutes. The flow path of nitrogen gas was switched from bubbling into the liquid to flowing into the space above the liquid, and the mixture was stirred while heating to 35 °C. After heating, 1.61 g of 3-methacryloxypropylmethyldimethoxysilane of formula 12 (Shin-Etsu Chemical Co., Ltd.) as a silane coupling agent was added and stirred for 30 minutes. Next, as a compound (monomer) of formula (2), 44.94 g of 2-methacryloyloxyethyl phthalate of formula 15 (Tokyo Kasei Kogyo Co., Ltd.) was dissolved in 150 g of pure water and 70 g of 10 wt% sodium hydroxide aqueous solution, then added to the reaction tank and stirred for 30 minutes. The mixture was then stirred for an additional 30 minutes while heating to 65 °C. Subsequently, 6.69 g of 2,2'-azobis[N-(2-carboxyethyl)-2-methylpropionamidine] n-hydrate (Wako Pure Chemical Industries, Ltd.) was dissolved in 30 g of pure water and added to the reaction tank. After addition, the mixture was stirred for 4 hours. After completion of the polymerization reaction, the particles were recovered with a magnet, and the supernatant was removed. The particles were then redispersed in 200 g of pure water and recovered with a magnet, and the supernatant was removed. This washing operation from redispersion in pure water to removal of the supernatant was repeated four more times, and the resulting particles were dispersed in pure water. In this manner a slurry in which the polymer-coated magnetic metal particles of Example 6 were dispersed was obtained.

In Examples 7-9, as shown in Table 3, polymer-coated magnetic metal particles were produced in the same manner as Example 6, except that the type of compound (monomer) of formula (2) was appropriately changed.

In Example 10, a polymer layer was formed on the surface of silica-coated particles produced in the same manner as Example 1. Specifically, 8.00 g of silica-coated particles (silica content 0.16 g) was mixed with 40 g of pure water and sonicated for 10 minutes. The sonicated suspension and 517.94 g of pure water were placed in a 1 L beaker, and bubbled with nitrogen gas at 0.1 L/min for 30 minutes. The nitrogen gas flow path was switched from bubbling into the liquid to flowing into the space above the liquid, and the mixture was stirred while heating to 35 °C. After heating, 1.84 g of 3-methacryloxypropylmethyldimethoxysilane of formula 12 (Shin-Etsu Chemical Co., Ltd.) as a silane coupling agent was added and stirred for 30 minutes. Next, as a compound (monomer) of formula (2), 30.8 g of 2-methacryloyloxyethyl phthalate (Tokyo Kasei Kogyo Co., Ltd.) was dissolved in 150 g of pure water and 55 g of 10 wt% sodium hydroxide aqueous solution and added to the reaction tank, then stirred for 30 minutes. The mixture was further stirred for 30 minutes while heating to 65 °C. Subsequently, 4.59 g of 2,2'-azobis[N-(2-carboxyethyl)-2-methylpropionamidine] n-hydrate (Wako Pure Chemical Industries, Ltd.) was dissolved in 20 g of pure water and added to the reaction tank. After addition, the mixture was stirred for 4 hours. After completion of the polymerization reaction, the particles were recovered with a magnet, and the supernatant was removed. The particles were then redispersed in 200 g of pure water and recovered with a magnet, and the supernatant was removed. This washing operation from redispersion in pure water to removal of the supernatant was repeated four more times, and the resulting particles were dispersed in pure water. In this manner, the slurry in which the polymer-coated magnetic metal particles of Example 10 were dispersed was obtained.

In Examples 11-14, as shown in Table 3, polymer-coated magnetic metal particles were produced in the same manner as Example 10, except that the type of compound (monomer) of formula (2) was appropriately changed.

In Example 15, as shown in Table 3, polymer-coated magnetic metal particles were produced in the same manner as Example 10, except that the silane coupling agent used for forming the polymer layer was changed to 2.05 g of 3-methacryloxypropylmethyldiethoxysilane of formula 13.

In Example 16, as shown in Table 3, polymer-coated magnetic metal particles were produced in the same manner as Example 10, except that the silane coupling agent used for forming the polymer layer was changed to 1.85 g of 3-acryloxypropyltrimethoxysilane of formula 5.

**[Table 4]**

| | Evaluation | | | | | | |
|---|---|---|---|---|---|---|---|
| | D50 [µm] | Biotin binding amount | Saturation magnetization σs [Am²/k_{g}] | Coercivity Hc | | **Carbon content** [%] | Storage stability retention rate (%) |
| | | | | [Oe] | [kA/m] | | |
| Example 6 | 1.1 | 258 | 134 | 19 | 1.5 | 0.2 | 84 |
| Example 7 | 1.2 | 292 | 132 | 18 | 1.4 | 0.2 | 89 |
| Example 8 | 1.2 | 292 | 131 | 18 | 1.4 | 0.2 | 89 |
| Example 9 | 1.1 | 258 | 132 | 19 | 1.5 | 0.2 | 77 |
| Example 10 | 2.3 | 217 | 188 | 5 | 0.4 | 1.0 | 69 |
| Example 11 | 2.7 | 250 | 193 | 5 | 0.4 | 0.6 | 60 |
| Example 12 | 2.5 | 267 | 196 | 5 | 0.4 | 0.6 | 63 |
| Example 13 | 2.4 | 275 | 193 | 5 | 0.4 | 0.7 | 61 |
| Example 14 | 2.5 | 250 | 194 | 5 | 0.4 | 0.6 | 53 |
| Example 15 | 2.3 | 225 | 190 | 5 | 0.4 | 0.9 | 48 |
| Example 16 | 2.2 | 250 | 189 | 5 | 0.4 | 0.9 | 47 |

As shown in Table 4, in Examples 6-9, similarly to Example 5, a high biotin binding amount, high saturation magnetization, and low coercivity were confirmed. In Examples 10-16, similarly to Example 1, a high biotin binding amount, high saturation magnetization, and low coercivity were also confirmed. In addition, in Examples 6-16, the thickness of the coating layer was confirmed to be comparable to that in Example 1.

Furthermore, in Examples 6-16, compared with Examples 1-5, the retention rate of the carried amount was higher, confirming superior storage stability. This is presumably because, in Example 6, for instance, a larger amount of silane coupling agent was used than in Example 1,which allowed the polymer layer to be formed more uniformly and thicker on the coating layer.

As described above, in polymer-coated magnetic metal particles, by forming the polymer layer to include a polymer constituted from a structural unit represented by formula (1) and an alkoxysilane having an acrylic group or a methacrylic group, it becomes easier to bind carrier substances, such as streptavidin, which can capture the target substance to be tested, thereby increasing the carried amount thereof. Moreover, by with a magnetic metal particle as the core, the proportion of magnetic metal in the polymer-coated magnetic metal particle can be increased, improving magnetic responsiveness and magnetic separability thereof.

## Claims

1. A polymer-coated magnetic metal particle comprising:
a magnetic metal particle;
a coating layer which is provided on a surface of the magnetic metal particle and is constituted from silicon oxide; and
a polymer layer which is provided on a surface of the coating layer and includes a polymer of a structural unit represented by formula (1) and alkoxysilane having an acrylic group or a methacrylic group. 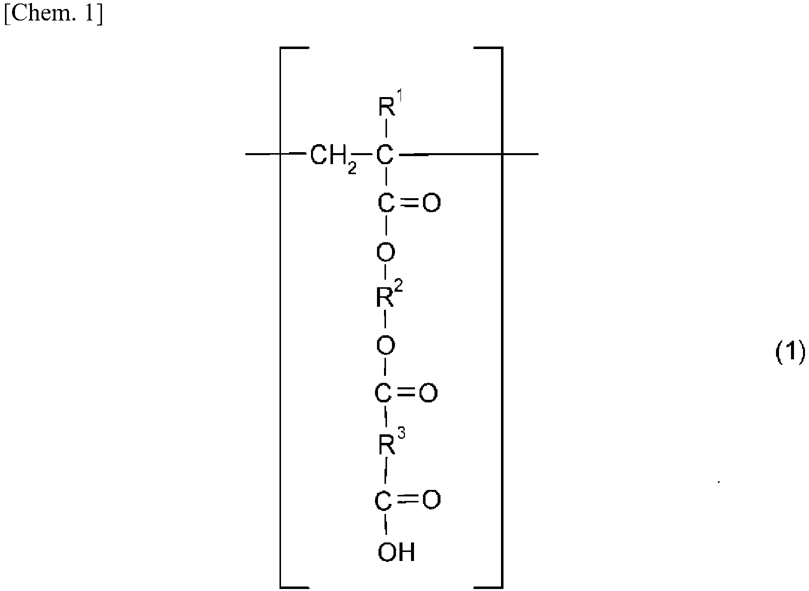 (in formula (1), R¹ represents a hydrogen atom or a methyl group, R² represents a linear or branched alkylene group having 2 or more and 6 or less carbon atoms, and R³ represents an alkylene group having 2 or more and 6 or less carbon atoms, a cyclohexylene group or a phenylene group).

2. The polymer-coated magnetic metal particle according to claim 1, wherein, in formula (1), R² is an ethylene group, and R³ is an alkylene group, a phenylene group or a cyclohexylene group.

3. The polymer-coated magnetic metal particle according to claim 2, wherein in formula (1), R³ is a phenylene group.

4. The polymer-coated magnetic metal particle according to claim 1 or 2, wherein the alkoxysilane has an acrylic group or a methacrylic group and an alkylene group having 3-6 carbon atoms.

5. The polymer-coated magnetic metal particle according to claim 4, wherein the alkoxysilane has an acryloxypropyl group or a methacryloxypropyl group.

6. The polymer-coated magnetic metal particle according to claim 1 or 2, wherein the magnetic metal particle is an iron particle or an iron-based alloy particle.

7. The polymer-coated magnetic metal particle according to claim 1 or 2, wherein saturation magnetization is100 Am²/kg or more and 210 Am²/kg or less.

8. The polymer-coated magnetic metal particle according to claim 1 or 2, wherein a 50% cumulative particle size by volume measured by a laser diffraction particle size distribution analyzer is 0.2-10 µm.

9. The polymer-coated magnetic metal particle according to claim 1 or 2, wherein carbon content is 0.5 mass% or more and 10 mass% or less.

10. The polymer-coated magnetic metal particle according to claim 1 or 2, wherein carbon content is 0.1 mass% or more and 10 mass% or less.

11. A method of producing a polymer-coated magnetic metal particle, the method comprising:
forming a coating layer constituted from silicon oxide on a surface of a magnetic metal particle; and
mixing the magnetic metal particle on which the coating layer is formed, water, and alkoxysilane having an acrylic group or a methacrylic group to obtain a mixture, then adding a compound represented by formula (2) to the mixture, and polymerizing the mixture to which the compound is added to form a polymer layer on the coating layer. 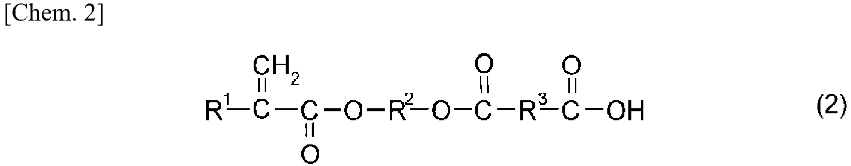 (in formula (2), R¹ represents a hydrogen atom or a methyl group, R² represents a linear or branched alkylene group having 2 or more and 6 or less carbon atoms, and R³ represents an alkylene group having 2 or more and 6 or less carbon atoms, a cyclohexylene group or a phenylene group).

12. The method of producing a polymer-coated magnetic metal particle according to claim 11, wherein, in formula (2), R² is an ethylene group, and R³ is an alkylene group, a phenylene group or a cyclohexylene group.

13. The method of producing a polymer-coated magnetic metal particle according to claim 12, wherein, in formula (2), R³ is a phenylene group.

14. The method of producing a polymer-coated magnetic metal particle according to claim 11 or 12, wherein the alkoxysilane has: an acrylic group or a methacrylic group; and an alkylene group having 3-6 carbon atoms.

15. The method of producing a polymer-coated magnetic metal particle according to claim 14, wherein the alkoxysilane has an acryloxypropyl group or a methacryloxypropyl group.

16. The method of producing a polymer-coated magnetic metal particle according to claim 11 or 12, wherein the magnetic metal particle is an iron particle or an iron-based alloy particle.

17. The method of producing a polymer-coated magnetic metal particle according to claim 11 or 12, wherein, in the forming of the polymer layer, a water-soluble azo polymerization initiator having a carboxyl group is used as a polymerization initiator.
